## Europäisches Patentamt

## European Patent Office

## Office européen des brevets

(11) Publication number: **0 182 401**
**B1**

## EUROPEAN PATENT SPECIFICATION

(45) Date of publication of patent specification: **19.07.89**

(21) Application number: **85201657.5**

(22) Date of filing: **10.10.85**

(51) Int. Cl.⁴: **A 61 K 39/00,** A 61 K 39/095, A 61 K 39/165, A 61 K 39/39

(54) Vaccines comprising immunogenic protein or peptide complexes.

(30) Priority: **19.10.84 NL 8403195**

(43) Date of publication of application: **28.05.86 Bulletin 86/22**

(45) Publication of the grant of the patent: **19.07.89 Bulletin 89/29**

(84) Designated Contracting States: **AT BE CH DE FR GB IT LI LU NL SE**

(56) References cited:
EP-A-0 037 931
EP-A-0 090 660
EP-A-0 109 942
FR-A-2 446 111

CHEMICAL ABSTRACTS, vol. 99, no. 7, 15th August 1983, page 386, no. 51586x, Columbus, Ohio, US; B. MOREIN et al.: "Protein subunit vaccines of parainfluenza type 3 virus: immunogenic effect in lambs and mice", & J. GEN. VIROL. 1983, 64(7), 1557-69

(73) Proprietor: **DE STAAT DER NEDERLANDEN VERTEGENWOORDIGD DOOR DE MINISTER VAN WELZIJN, VOLKSGEZONDHEID EN CULTUUR**
**P.O. Box 439**
**NL-2260 AK Leidschendam (NL)**

(72) Inventor: **Beuvery, Eduard Coen**
**Kerkstraat 66**
**NL-4132 BG Vianen (NL)**
Inventor: **Teerlink, Tom**
**Donkeregaarde 53**
**NL-3436 ZD Nieuwegein (NL)**
Inventor: **van Wezel, Antonius Ludovicus**
**Kruislaan 18**
**NL-3721 AM Bilthoven (NL)**

(74) Representative: **van der Beek, George Frans, Ir. et al**
**Nederlandsch Octrooibureau Scheveningseweg 82 P.O. Box 29720**
**NL-2502 LS 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# EP 0 182 401 B1

**Description**

The invention relates to vaccines comprising immunogenic complexes derived from antigenic proteins or peptides.

Background of the invention

The use of purified bacterial and viral membrane proteins or synthetic peptides and of proteins prepared by recombinant DNA techniques as antigens for the immunization of humans and animals offers certain advantages over the use of whole cells or parts thereof. Namely, the use of purified proteins allows exclusion of components causing undesirable, for example pyrogenic, reactions. Further, the proteins may be selected such that they possess—as far as possible exclusively—the desirable immunogenic properties, and, moreover, do not show isogenic variation. Highly purified membranes proteins, especially bacterial outer membranes proteins and viral envelope glycoproteins have, however, the disadvantage that they generally possess only low immunogenic activity.

It is known that antigenic proteins may form complexes with detergents. The detergents serve to solubilize the proteins. Some of these protein detergent complexes have been used as experimental immunogens. Thus, in J. Gen. Virol. *64*, 1557—1569 (1983) protein subunit vaccines of parainfluenza type 3 virus are described, including vaccines containing Triton X-100 complexes. The article does not indicate any advantage of the detergent complexes over the detergent-free proteins.

EP—A—90660 relates to a method of separating and purifying protein I from the principal outer membrane of *Neisseria gonorrhoeae* and to vaccines produced therefrom. The method involves the use of a specific solubilizing agent comprising a detergent. All detergents are removed very carefully before use of the protein in a vaccine.

FR—A—2 446 111 (French Patent Application 7901301) describes a process for extracting antigenic complexes from micro-organisms wherein sodium deoxycholate, lysozyme and sodium EDTA are used as extraction agents. The sodium deoxycholate remains in the vaccine.

Summary of the invention

It was found that the immunogenic activity of antigenic proteins and peptides is surprisingly improved if they are in the form of adsorbed detergent complexes. The improvement of the immunogenic activity obtained by complexing the proteins or peptides with a detergent is increased far more strongly by adsorption of the detergent complex onto an adsorbent, than would be expected from the effect observed when the uncomplexed proteins or peptides are adsorbed onto the adsorbent. So, the combination of detergent and adsorbent results in a true synergistic effect.

Consequently, the invention relates to a vaccine comprising an antigenic protein or peptide, a detergent, and an adsorbent.

It is to be understood that the expression "antigenic protein or peptide" includes antigenic proteinaceous products, such as glycoproteins and polysaccharide-protein conjugates.

Detailed description and preferred embodiments

The invention is mainly elucidated by adsorbed detergent complexes of outer membrane proteins derived from *Neisseria gonorrhoeae, Neisseria meningitidis* and *Haemophilus influenzae*, as well as by adsorbed detergent complexes of measles glyco-protein. The invention is not limited thereto, however.

Good results have been obtained especially with porine proteins derived from gonococci or meningococci. These outer membrane proteins have advantages over other immunogenic proteins, because they are present in the membrane in large amounts. For example, the porine protein PI of *N. gonorrhoeae* amounts to about 50% of the total amount of proteins present on the outer membrane. Further, it is advantageous that PI is present in all gonococcal strains and shows a minimum isogenic variation. Also part of the molecule protrudes from the membrane, so that it is capable of reacting with antibodies.

It has appeared that antisera of serotype 1 gonococcal PI, 5 and 9 show a considerable cross reactivity. Therefore, it is advantageous to incorporate into a vaccine adsorbed protein detergent complexes in which the protein component is a mixture of gonococcal PI's derived from a number of different serotypes, for example of serotypes 1, 5 and 9. The result will be that the vaccine will induce formation of antibodies which react with PI of all nine serotypes.

The nature of the detergent which is present in the vaccines according to the invention is not critical. The detergent may be a Zwitterionic, kationic, anionic or non-ionic detergent.

Examples of suitable Zwitterionic (betaine) detergents are the Zwittergents, such as Zwittergent 3—14, also called Z3—14, the chemical name of which is 3 - (N - tetradecyl - N,N - dimethyl - ammonio) - propane - 1 - sulphonate, and derivates thereof having a shorter or longer hydrocarbon chain, such as Z3—10, Z3—12, Z3—16, and Z3—18. Further, N - lauroyl - sarcosine (sarcosyl) may be mentioned as a suitable Zwitterionic detergent.

Cetrimonium bromide (CTAB) is an example of a suitable kationic detergent, and dioctyl sodium sulfosuccinate (DONS), sodium dodecyl sulphate (SDS), and sodium deoxycholate (DOC) may be mentioned as effective anionic detergents.

2

EP 0 182 401 B1

Suitable neutral or non-ionic detergents are, for example, n-octyl glucoside, Triton X-100, Tween 20, Tween 80, Brij 52, Brij 58, and Myrj 45.

The ratio of antigenic protein or peptide to detergent influences the immunogenic properties of the adsorbed protein or peptide/detergent complex. A general rule for this ratio cannot be given, as the ratio giving the highest immunogenicity appears to vary dependent on the particular protein or peptide and on the particular detergent used. Optimum ratios may be determined easily by testing the immunogenic activity of a series of vaccines containing antigen and detergent in varying ratios. For example, in case of gonococcal PI outer membrane protein the immunogenic activity in mice appeared to be optimal at a protein; detergent ratio (w/w) of about 1:5 to 1:2.5 when the detergent was Z3—14, at a ratio of 1:5 when the detergent was octyl glucoside or DOC, and at a ratio of 1:1 to 1:0.6 when the detergent was CTAB.

When the antigenic protein in the vaccine according to the invention is a gonococcal outer membrane protein PI or a mixture of gonococcal OMP PI's, best results have been obtained with Z3—10, Z3—14, N-lauroylsarcosine, sodium deoxycholate, Triton X-100, Tween 20, and Brij 58 as detergents. Preferred detergents to be used with Group B, type 15 (P1.16) meningococcal outer membrane protein combination are Z3—14 and sodium deoxycholate.

The adsorbents to be used in the vaccines of the invention are adsorbents generally used in vaccines. Examples of such adsorbents are aluminium hydroxide, aluminium phosphate, and calcium phosphate. Aluminium phosphate is preferred.

The following examples serve to illustrate the invention in further detail.

Example 1

Gonococcal strains B2 (serotype 1), C3 (serotype 5) and F6 (serotype 9) were grown in the usual way. The cultures were inactived by heating at 56°C during 30 minutes. The bacteria were lyophilized after centrifugation. The cell-free culture liquid was used for the preparation of outer membrane complexes (OMC), and purified PI was prepared from the lyophilized bacteria.

Preparation of the outer membrane complexes.

The cell-free culture liquid was concentrated 80-fold on an Amicon hollow fiber cartridge (H10P10). The concentrate was centrifuged (10,000×g; 20 min) to remove any residual bacteria. The OMC was centrifuged (2h; 100,000×g), the pellet was suspended in 300 mM NaCl-50 mM Tris, pH 7.2, centrifuged again (2h at 100,000×g) and the pellet finely suspended in distilled water to obtain a protein concentration of 2 mg/ml.

Purification of PI.

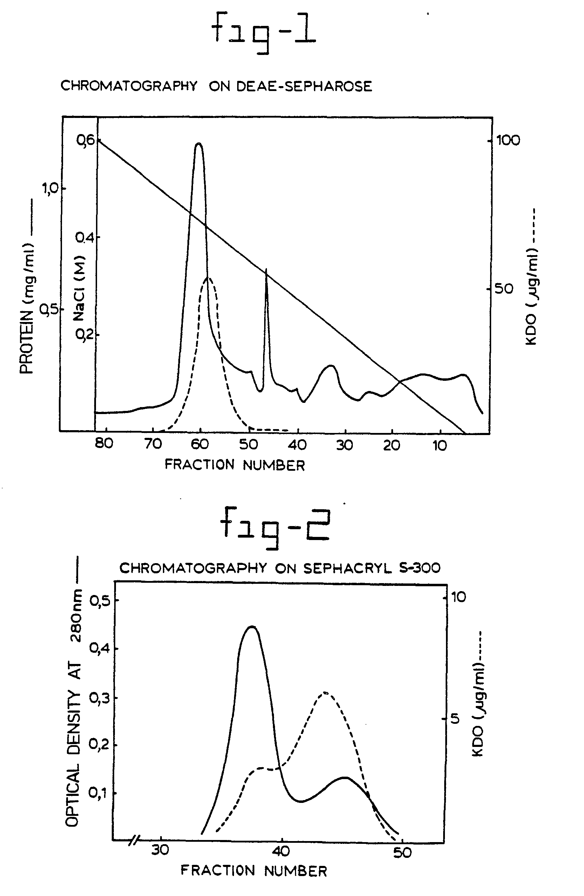

The isolation procedure was based on the procedure which has been used to Blake and Gotschlich for the isolation of protein II (J. Exp. Med. *159*, 452—462 (1984)). Lyophilized gonococci (2.5 g dry weight) were extracted with 2.5 g of 3 - (N - tetradecyl - N,N - dimethylammonio) - propane - 1 - sulphonate (Z3—14) in 0.5 M calcium chloride at pH 4.0 in a total volume of 250 ml. After 1 h intact cells and debris were removed by centrifugation (20 min., 10,000×g). If necessary the pH of the supernatant was readjusted to 4.0 with dilute HCl, and ethanol was added to a concentration of 20%. After 30 min. precipitated material was removed by centrifugation (20 min., 10,000×g). The supernatant was concentrated to 150 ml by ultrafiltration (Amicon hollow fibre H1D×50); 150 ml 50 mM Tris-10 mM EDTA-0.05% (w/v) Z3—14, pH 8.0 (buffer A) was added and the volume was reduced 2-fold; this procedure was repeated 5 times to ensure complete removal of $CaCl_2$ and ethanol. Hereafter the protein solution was applied to a DEAE-Sepharose column (50×1.8 cm) equilibrated with buffer A. The proteins were eluted with a linear gradient from 0.0 to 0.6 M NaCl in buffer A (2×400 ml), at a flow rate of 50 ml/h. The results are given in Figure 1. Fractions were analyzed by SDS-PAGE and PI containing fractions were pooled. This product is called partially purified PI.

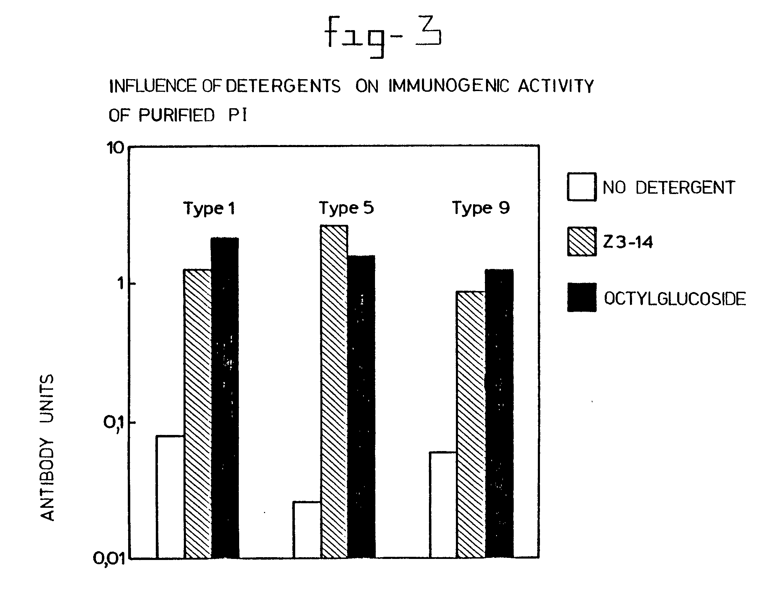

The partially purified PI was applied to a Sephacryl S-300 column (80×3.6 cm), previously equilibrated with 50 mM Tris-200 mM NaCl-10 mM EDTA-0.05% (w/v) Z3—14, pH 7.2. The column was eluted at a flow rate of 50 ml/h. The results are given in Figure 2. PI containing fractions were pooled and stored at 4°C with 0.02% (w/v) sodium azide as a preservative. The product obtained in this stage is called purified PI.

Analytical methods.

Protein was determined by the method of Lowry as described in J. Biol. Chem. *193*, 265—275 (1951), and modified by Peterson (Anal. Biochem. *83*, 346—356 (1977)), with bovine serum albumin as a standard. Ketodesoxyoctonoate (KDO) was assayed following the procedure of Karkhanis et al. (Anal. Biochem. *85*, 595—601 (1978)) using KDO as a standard. SDS-PAGE was performed as described by Laemmli (Nature *227*, 680—685 (1970)).

Biological activities.

For the pyrogenicity test in rabbits the protocol of European Pharmacopoeia II, pages 53—61 (1971) was used.

ELISA procedure.

For the termination of antibody levels of serum samples, microtiter plates were coated with OMC diluted with 0.15 M NaCl to a protein concentration of 10 µg/ml. The subsequent steps of the ELISA were

3

performed as described in Infect. Immun. *40*, 369—380 (1983). The IgG antibody levels are expressed as arbitrary antibody units.

Preparation of protein-detergent complexes.

Purified PI in buffer containing Z3—14 was precipitated with 80% ethanol. The protein pellet was washed once with 80% ethanol to remove residual Z3—14. To the protein pellet (100 μg) was added 0.05 ml of a 10 mg/ml solution of detergent in 0.9% (w/v) NaCl; after an incubation time of 16 h, 0.95 ml of a 0.9% (w/v) NaCl solution was added and the samples were briefly sonicated (30 sec., 60 W). Before immunization the samples were diluted to the following final concentrations: protein: 5 μg/ml; detergent: 25 μg/ml; NaCl: 9 mg/ml. To some samples an AlPO$_4$ suspension was added to a final concentration of 0.5 μg/ml.

Immunization of mice.

Groups consisting of eight random bred mice were injected intraperitoneally (i.p.) with 0.5 ml of the PI-detergent complexes. After four weeks the mice were bled and the antibody levels of the pooled sera were determined by ELISA.

Results

Purification of serotype 1, 5 and 9 PI.

PI was effectively solubilized from a bacterial suspension by Z3—14 in the presence of CaCl$_2$ at pH 4.0. One g of Z3—14 per g of bacteria (dry weight) was used for solubilization of approximately 100 mg of protein.

After a 20% ethanol precipitation step, ethanol and CaCl$_2$ were removed from the supernatant by ultrafiltration. The protein was subsequently applied to a DEAE-Sepharose column and eluted with a NaCl gradient (Figure 1). Partially purified PI was eluted as a symmetrical peak at a molarity of 0.4. The elution profile of lipopolysaccharide (LPS) was also determined by an assay for KDO. Figure 1 shows that the top of the KDO peak precedes that of the PI peak. However, a complete separation of both components could not be achieved on this column. Subsequent gel permeation chromatography on Sephacryl S-300 provided a further resolution of both components (Figure 2). PI was eluted as a symmetrical peak (fractions 34 to 38), followed by a second peak containing some low molecular weight proteins and the bulk of the lipopolysaccharide, SDS-PAGE analysis revealed that the first peak was essentially pure PI. Starting with 2.5 g (dry weight) of bacteria, the PI yield was usually between 20 and 50 mg. Table A gives a summary of the results of a typical purification procedure, including mention of recoveries for protein and KDO.

TABLE A
Purification of protein I (serotype 1), starting from
2.5 g (dry weight) of bacteria.

| Purification step | protein | | KDO | | KDO/protein |
|---|---|---|---|---|---|
| | total (mg) | recovered (%) | total (mg) | recovered (%) | μg/mg |
| Z3—14 extract | 272 | 100 | 4,35 | 100 | 16 |
| 20% ethanol precipitation | 85 | 31 | 2,80 | 64 | 33 |
| DEAE-Sepharose | 41 | 15 | 0,69 | 16 | 17 |
| Sephacryl S-300 | 24,4 | 8,9 | 0,17 | 3,9 | 7 |

Analysis and biological activity of purified PI.

The KDO content of partially purified PI was between 15 and 50 μg KDO/mg protein. After further purification on Sephacryl S-300 this value was reduced to 6 to 7 μg KDO/mg protein. Assuming a molecular weight for PI of 34,000, this amounts to a molar ratio of KDO to protein of 1. Purified PI passed the pyrogenicity test at a dose of 25 ng of protein ker kg of rabbit body weight.

Immunogenic activity of purified PI.

The induction of antibodies in mice by PI, present in three different conditions was studied. These conditions were (a) purified PI in Z3—14, (b) a preparation in which Z3—14 was replaced by octyl glucoside, and (c) PI without any detergent. Pooled serum samples, obtained after one injection, were tested in an ELISA in which the homologous OMC served as antigen. Figure 3 shows the relative antibody levels of the various samples. It is evident that both detergents enhanced the immunogenic activity of purified PI considerably. No significant differences between the preparations containing Z3—14 and octyl glucoside were observed. However, in the absence of detergents, PI was a poor immunogen. Then, the influence of other detergents on the immunogenic activity of PI was investigated. Table B shows the results of the immunization experiment.

TABLE B

Effect of addition of detergent and $AlPO_4$ on the immunogenic potency of gonococcal PI outer membrane protein in mice.

| detergent | antibody units | |
|---|---|---|
| | with $AlPO_4$ | without $AlPO_4$ |
| none | 6,3 | 0,4 |
| Z3—10 | 55,4 | n.t.* |
| Z3—12 | 30,4 | n.t. |
| Z3—14 | 45,3 | 6,9 |
| Z3—16 | 17,5 | n.t. |
| Z3—18 | 32,3 | n.t. |
| sarcosyl | 85,7 | n.t. |
| CTAB | 14,8 | 1,0 |
| DONS | 26,4 | 1,4 |
| SDS | 36,8 | n.t. |
| DOC | 52,0 | n.t. |
| octyl glucoside | 38,8 | 13,6 |
| Triton X-100 | 62,1 | n.t. |
| Tween 20 | 48,4 | n.t. |
| Tween 80 | 37,0 | n.t. |
| Brij 52 | 17,1 | n.t. |
| Brij 58 | 45,9 | n.t. |
| Myrj 45 | 27,1 | n.t. |

*n.t.=not tested

Composition immunogen: protein 5 µg/ml
detergent 25 µg/ml
$AlPO_4$ 500 µg/ml
NaCl 9 mg/ml

dose: 0,5 ml i.p.
8 mice per group.

All detergents tested enhanced the immunogenic activity of Pi. This effect was synergistically increased when the protein-detergent complexes were adsorbed to $AlPO_4$.

The influence of the protein-detergent ratio was studied with four detergents (see Table C).

TABLE C

Influence of protein-detergent ratio on immunogenic potency of
adsorbed gonococcal PI outer membrane protein in mice.

| detergent | ratio (protein/detergent (w/w)) | | | |
|---|---|---|---|---|
| | 1:5 | 1:2.5 | 1:1.25 | 1:0.62 |
| | antibody units | | | |
| Z3—14 | 170.8 | 174.5 | 170.5 | 108.7 |
| octyl glucoside | 116.2 | 74.8 | 44.9 | 27.8 |
| DOC | 147.4 | 101.5 | 72.5 | 97.3 |
| CTAB | 31.9 | 79.9 | 77.7 | 99.0 |

control without detergent: 24.2

Composition immunogen: PI protein: 5 µg/ml
detergent: 3.12—25 µg/ml
$AlPO_4$: 500 µg/ml
NaCl: 9 mg/ml

dose: 0.5 ml i.p.
8 mice per group

At the lowest detergent concentration tested all four detergents enhanced the antibody response. In the case of Z3—14, octyl glucoside and deoxycholate the stimulating effect increased at higher detergent levels. With the detergent CTAB the opposite effect was observed.

Example 2

Meningococcal strain H44/76 (B:15:P1.16 (Rev. Infect. Dis. 7, 504—510 (1985)) was cultivated in a fermentor. The culture was inactivated by heating at 56°C for 30 min. After centrifugation the bacteria were pooled and lyophilized. The cell-free culture liquid was used for the preparation of OMC.

Preparation of OMC and purification of the combination of class 1 and 3 OMP's.

The preparation of OMC and the purification of the combination of class 1 and 3 OMP's were performed according to the procedures presented in Example 1.

Analytical methods and biologic activity.

The analytical methods and the measurement of the pyrogenic activity were as described in Example 1.

Assay of antibodies.

For both of the ELISA technique and the Western blotting technique, OMC was used as antigen for the assay of antibodies. The ELISA technique was performed as described in Example 1. The Western blotting technique was carried out by transferring the various outer membrane proteins to nitrocellulose paper and consecutive incubations with serum samples from mice, with peroxidase conjugated antibodies to mouse IgG 1+2 and with substrate.

Immunization of mice.

Groups of eight random bred mice were injected i.p. with the OMP combination present in various detergent micelles or as aggregate both in free condition and adsorbed to $AlPO_4$. The preparation of the various OMP-detergent micelles was carried out as described in Example 1.

Results
Characterization of the OMP combination.

OMC and the purified OMP combination were analysed by SDS-PAGE, which revealed that the purified OMP combination contained—in addition to class 1 and 3 OMP's—small amounts of a 77Kd OMP and of class 4 OMP.

The KDO content of the purified OMP combination was about 50 µg KDO per mg of protein. The combination passed the pyrogenicity test in a dose of 25 ng of protein per kg of rabbit body weight.

Immunogenic activity of the purified OMP combination.

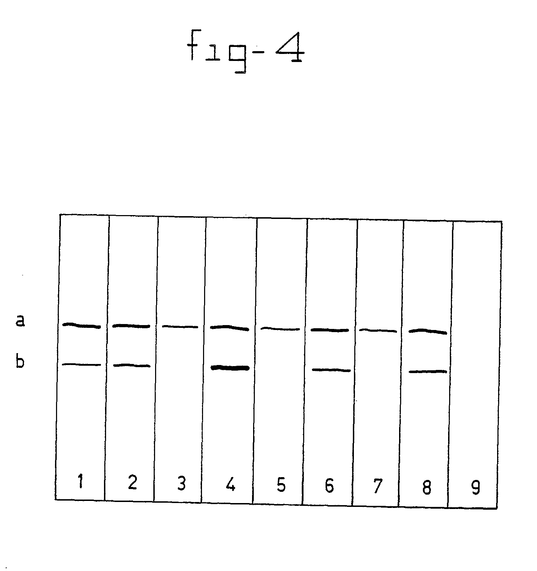

Groups of mice were injected.with the OMP combination in different conditions (see Table D). Pooled serum samples obtained after one injection were tested in ELISA and in Western blotting. The results are shown in Table D (ELISA) and in Figure 4 (Western blotting). Table D shows a small stimulation of the immunogenic activity of the combination by the various detergents and a substantial stimulation by detergent plus adsorbent.

TABLE D
Effect of addition of detergent and $AlPO_4$ on the immunogenic
potency of a meningococcal group B, type 15 (P1.16)
outer membrane protein combination in mice. The antibodies
were assayed with the ELISA technique.

| | antibody units | |
| detergent | with $AlPO_4$ | without $AlPO_4$ |
| --- | --- | --- |
| none | 8.7 | n.t.* |
| Z3—14 | 11.9 | 1.2 |
| DOC | 18.0 | 1.3 |
| octyl glucoside | 12.7 | 2.2 |
| Triton X-100 | 10.5 | n.t. |

*n.t.=not tested.
Composition of immunogen and dosage, see Table B.

In Figure 4, class 1 and 3 OMP's were blotted as bands at positions a and b, respectively. The figure shows that the combination of DOC and $AlPO_4$ was, unlike the other combinations, able to provoke antibodies to class 3 OMP after injection together with the OMP combination.

The lanes in Figure 4 relate to the following products:
1. Protein+$AlPO_4$
2. Protein+Z3—14+$AlPO_4$
3. Protein+Z3—14
4. Protein+DOC+$AlPO_4$
5. Protein+DOC
6. Protein+octyl glucoside+$AlPO_4$
7. Protein+octyl glucoside
8. Protein+Triton X-100+$AlPO_4$
9. Control group (a non-immunized group).

Example 3

Meningococcal strain MC19 (J. infect. Dis. *129*, 147—153 (1974)) and *Haemophilus influenzae* type b strain Amsterdam (provided by Dr. L. van Alphen, University of Amsterdam) were cultivated in a fermentor. The cultures were inactivated by heating at 56°C for 30 min. After centrifugation the cell-free culture liquid was collected from the meningococcal culture and the bacteria from the *H. influenzae* culture. The *H. influenzae* bacteria were lyophilized.

Preparation of purified meningococcal group $C^-$ polysaccharide, *H. influenzae* type b OMP and polysaccharide-OMP conjugate.

Purified meningococcal group $C^-$ polysaccharide (PS) was prepared from the cell-free culture liquid as described in WHO. Techn. Rep. Ser. *594*, 50—75 (1967). *H. influenzae* type b OMP was purified according to the method described in Example 1. The conditions for conjugation of group $C^-$ PS with *H. influenzae* type b in Zwittergent 3—14 and the purification of the conjugate was carried out as described by Beuvery et al. in Infect. Immun. *40*, 39—45 (1983) with the difference that, after conjugation, the reaction was not quenched.

Analytical methods.

Protein was determined as described in Example 1. Group $C^-$ PS was assayed by the method of Svennerholm (Biochem. Biophys. Acta *24*, 604 (1957)) with N-acetylneuraminic acid as standard.

Assay of antibodies.

Antibodies to group C⁻ PS were assayed in ELISA as described in Infect. Immun. *40*, 369—380 (1983).

Immunization of mice.

Groups of mice were injected as described in Example 1 with the conjugate combined with and without detergent and with and without $AlPO_4$. The immunogens were prepared as described in Example 1. The dose amounted to 2.5 µg of group C⁻ PS.

Results

Characterization of the separate components of the conjugate and of the conjugate.

Purified group C⁻ PS met the WHO minimum requirements for group C⁺ PS (WHO Tech. Rep. Ser. *594*, 50—75 (1967); *658*, 175—184 (1981)) except that no O-acetyl groups could be detected. *H. influenzae* cell envelopes (provided by Dr. van Alphen) and purified OMP were analyzed by SDS-PAGE. Purified OMP contained proteins bc (J. Bacteriol. *155*, 878—885 (1983)). The conjugate was composed of about equal amounts of PS and protein.

Immunogenic activity of the PS component of the conjugate

Groups of mice were injected with the conjugate in different conditions (see Table E). Pooled serum samples obtained after one injection were tested in ELISA (Table E). The results show that the combination of detergent and adsorbent acted synergistically on the antibody response to PS component of the conjugate.

TABLE E

Effect of detergent and $AlPO_4$ addition on the immunogenic potency of Group C meningococcal polysaccharide/*Haemophilus influenzae* outer membrane protein conjugate in mice.

| | antibody units | |
|---|---|---|
| | with | without |
| Detergent | $AlPO_4$ | $AlPO_4$ |
| — | 0.5 | 0.7 |
| Z3—14 | 3.1 | 0.1 |
| Sarcosyl | 2.9 | 0.9 |
| DOC | 3.3 | 0.4 |
| CTAB | 2.5 | 1.2 |
| control | <0.1 | <0.1 |

Composition immunogen: see Table B.

Example 4

A plaque variant of the Edmonston strain measles virus was produced in Vero cells in a microcarrier culture. The virus suspension was filtered and concentrated by Amicon hollow fiber (H10×100) ultrafiltration. Viral glycoproteins were solublized in 10 mM Tris-HCl pH 7.8, 150 mM NaCl, 600 mM KCl, 0.1 mM $MgCl_2$, 2% (v/v) Triton X-100 and 1 mM phenylmethylsulfonyl fluoride (J. gen. Virol. *65*, 355—366 (1984)). After solubilization and ultracentrifugation (100,000×g, 60 min.) fusion protein was removed from the supernatant by affinity chromatography. To this end monoclonal antibodies to fusion proteins were covalently attached to Sepharose 4B. The fusion protein was desorbed by using 5 M $NH_4SCN$ in 20 mM Tris-HCl pH 7.8, 1 mM EDTA and 0.1% (w/v) octyl glucoside. Pooled fractions of the $NH_4SCN$ eluate, containing purified fusion protein, were dialyzed. The dialyzed fractions were assayed for protein (Anal. Biochem. *72*, 248 (1976)), analyzed by SDS-PAGE (see Example 1) and stored at −70°C.

ELISA procedure

Serum samples obtained from mice after injection with the various fusion protein containing preparations were assayed in ELISA. Microtiter plates were coated with purified, inactivated measles virus which was obtained after the following steps: a) filtration of the viral suspension, b) concentration of the suspension, c) gel filtration of the suspension on Sepharose CL-6B, d) inactivation of the viral suspension with 3-hydroxypropionic acid lactone and freeze-drying. The reconstituted suspension was tested for its potential to lyse erythrocytes. The results of this experiment show that the suspension was fully active in lysis of erythrocytes.

Immunization of mice.

The immunization of groups of mice was performed as described in Example 1. The mice were injected with the fusion protein present in Z3—14 micelles or as an aggregate in plain and in adsorbed condition. The immunogens were prepared as described in Example 1. The injected amount was 2.5 µg of protein.

Results

Characterization of the purified fusion protein.

SDS-PAGE analysis of the viral suspension, supernatant after Triton X-100 treatment and purified fusion protein revealed that the purified fusion protein preparation was composed almost exclusively of this protein. No haemagglutinin could be detected.

Immunogenic activity of the purified fusion protein.

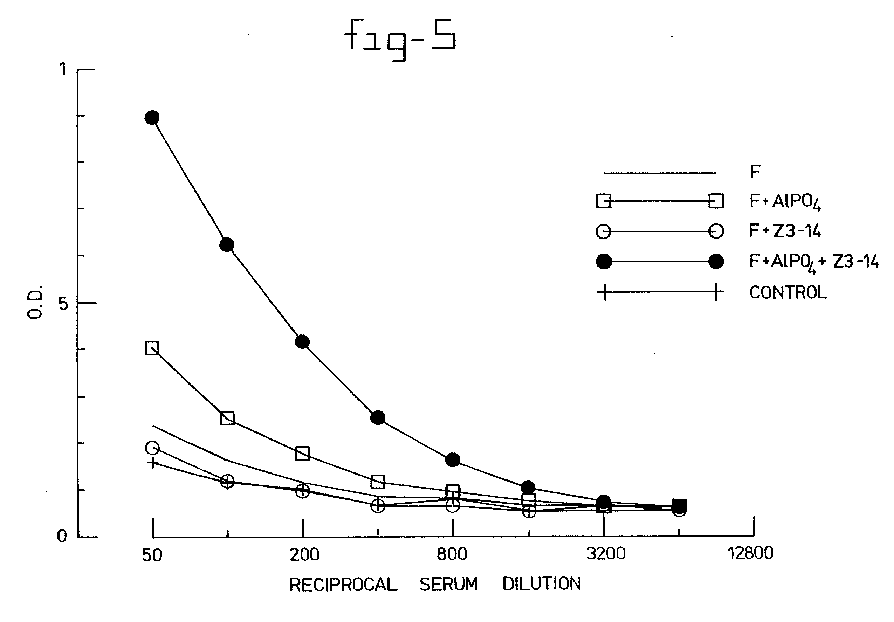

Groups of mice were injected with purified fusion protein and fusion protein as a Z3—14 micelle in combination with AlPO$_4$ and without adsorbent. Pooled serum samples obtained after one injection were tested in ELISA. The results of this test are presented in Figure 5 and show that the combination detergent and adsorbent acted synergistically on the antibody response by the fusion protein. In Figure 5 "F" means the fusion protein.

**Claims**

1. A vaccine comprising one or more antigenic proteins or peptides, a detergent and an adsorbent.

2. The vaccine of Claim 1 in which the antigenic protein is gonococcal outer membrane protein Pl.

3. The vaccine of Claim 1 in which the antigenic protein is a mixture of gonococcal Pl's derived from a number of different serotypes.

4. The vaccine of Claim 1, in which the antigenic protein is a group B, type 15 meningococcal outer membrane protein combination.

5. The vaccine of Claim 1, in which the antigenic protein is a meningococcal group C polysaccharide/ *Haemophilus influenzae* outer membrane protein conjugate.

6. The vaccine of Claim 1, in which the antigenic protein is a measles virus fusion protein.

7. The vaccine of Claim 1 in which the detergent is a Zwitterionic, kationic, anionic or non-ionic detergent.

8. The vaccine of Claim 2 or 3, in which the detergent is selected from the group consisting of Zwittergent® Z3—10 (3 - (N - decyl - N,N - dimethyl - ammonio) - propane - 1 - sulfonate), Zwittergent® Z3—14 (3 - (N - tetradecyl - N,N - dimethyl - ammonio) - propane - 1 - sulfonate), N - lauroylsarcosine, sodium deoxycholate, Triton® X-100, Tween® 20, and Brij® 58.

9. The vaccine of Claim 4, in which the detergent is selected from the group consisting of Zwittergent Z3—14 and sodium deoxycholate.

10. The vaccine of Claim 1, in which the adsorbent is aluminium phosphate, aluminium hydroxide or calcium phosphate.

11. The vaccine of Claim 10, in which the adsorbent is aluminium phosphate.

**Patentansprüche**

1. Impfstoff, enthaltend ein oder mehrere entigene Proteine oder Peptide, ein Detergens und ein Adsorptionsmittel.

2. Impfstoff nach Anspruch 1, worin das antigene Protein gonokokkales äußeres Membranprotein Pl ist.

3. Impfstoff nach Anspruch 1, worin das antigene Protein eine Mischung aus gonokokkalen Pl's, abgeleitet aus einer Anzahl verschiedener Serotypen, ist.

4. Impfstoff nach Anspruch 1, worin das antigene Protein eine meningokokkale äußere Membranproteinkombination der Gruppe B, Typ 15 ist.

5. Impfstoff nach Anspruch 1, worin das antigene Protein ein äußeres Membraneproteinkonjugat von meningokokkalem Gruppe C Polysaccharid/*Haemophilus influenzae* ist.

6. Impfstoff nach Anspruch 1, worin das antigene Protein ein Masernvirus-Fusionprotein ist.

7. Impfstoff nach Anspruch 1, worin das Detergent ein zwitterionisches, Kationisches, anionisches oder nicht ionisches Detergens ist.

8. Impfstoff nach Anspruch 2 oder 3, worin das Detergens aus der aus Zwittergent®Z3—10 (3 - (N - Decyl - N,N - dimethylammonio) - propan - 1 - sulfonat), Zwittergent®Z3—14, (3 - (N - tetradecyl - N,N - dimethylammonio) - propan - 1 - sulfonat), N - Lauroylsarcosin, Natriumdeoxycholat, Triton®X-100, Tween®20 und Brij®58 bestehenden Gruppe ausgewählt ist.

9. Impfstoff nach Anspruch 4, worin das Detergens aus der auz Zwittergent Z3—14 und Natriumdeoxycholat bestehenden Gruppe ausgewählt ist.

10. Impfstoff nach Anspruch 1, worin das Adsorptionsmittel Aluminumphosphat, Aluminiumhydroxid oder Calciumphosphat ist.

11. Impfstoff nach Anspruch 10, worin das Adsorptionsmittel Aluminiumphosphat ist.

**Revendications**

1. Vaccin contenant un(e) ou plusieurs protéines ou peptides antigéniques, un détergent et un adsorbent.

2. Vaccin de la revendication 1, dans lequel la protéine antigénique est la PI de protéine de membrane extérieure gonococcique.

3. Vaccin de la revendication 1, dans lequel la protéine antigénique est un mélange de PI gonococciques provenant d'un certain nombre de sérotypes différents.

4. Vaccin de la revendication 1 dans lequel la protéine antigénique est une combinaison de protéines de membrane extérieure méningococcique groupe B, type 15.

5. Vaccin de la revendication 1, dans lequel la protéine antigénique est un conjugué polysaccharide méningococcique groupe C/protéine de membrane extérieure *d'Haemophilus influenzae*.

6. Vaccin de la revendication 1 dans lequel la protéine antigénique est une protéine de fusion de virus de la rougeole.

7. Vaccin de la revendication 1 dans lequel le détergent est un détergent Zwitterionique, cationique, anionique ou non-ionique.

8. Vaccin de la revendication 2 ou 3, dans lequel le détergent est choisi dans le groupe constitué par le Zwittergent® Z3—10 (3 - (N - décyl - N,N - diméthylammonio)propane - 1 - sulfonate), le Zwittergent® Z3—14 (3 - (N - tétradécyl - N,N - diméthyl - ammonio) - propane - 1 - sulfonate), la N - lauroylsarcosine, le désoxycholate de sodium, le Triton® X-100, le Tween® 20 et le Brij® 58.

9. Vaccin de la revendication 4, dans lequel le détergent est choisi dans le groupe constitué par le Zwittergent Z3—14 et le désoxycholate de sodium.

10. Vaccin de la revendication 1, dans lequel l'adsorbant est le phosphate d'aluminium, l'hydroxyde d'aluminium ou le phosphate de calcium.

11. Vaccin de la revendication 10, dans lequel l'adsorbent est le phosphate d'aluminium.

EP 0 182 401 B1

fig-1

CHROMATOGRAPHY ON DEAE-SEPHAROSE

fig-2

CHROMATOGRAPHY ON SEPHACRYL S-300

1

# fig-3

INFLUENCE OF DETERGENTS ON IMMUNOGENIC ACTIVITY
OF PURIFIED PI

ANTIBODY UNITS

Type 1    Type 5    Type 9

☐ NO DETERGENT

▨ Z3-14

■ OCTYLGLUCOSIDE

EP 0 182 401 B1

# fig-4

fig-5

F
F+AlPO$_4$
F+Z3-14
F+AlPO$_4$+Z3-14
CONTROL

RECIPROCAL SERUM DILUTION

O.D.

EP 0 182 401 B1